# FASCICULE DE BREVET EUROPEEN

(11) **EP 1 330 980 B1**
(45) Date de publication et mention de la délivrance du brevet: **21.06.2006**
(21) Numéro de dépôt: 03290151.4
(22) Date de dépôt: 22.01.2003
(51) Int. Cl.: A61B 5/0424

(54) **Enregistreur de signaux physiologiques, notamment de type Holter, avec un dispositif de contrôle de la continuité d'un cable**
Gerät zur Registrierung von physiologischen Signalen, insbesondere vom Typ Holter, mit Kabeldurchgangsprüfgerät
Apparatus for recording physiological signals, in particular of the Holter type, with cable continuity testing

(30) Priorité: 23.01.2002 FR 0200817
(43) Date de publication de la demande: 30.07.2003
(73) Titulaire: ELA MEDICAL, 92541 Montrouge (FR)
(72) Inventeur: Faisandier, Yves, 75116 Partis (FR)
(74) Mandataire: Dupuis-Latour, Dominique

(56) Documents cités:
- EP-A- 0 182 197
- EP-A- 0 570 101
- EP-A- 0 711 530
- US-A- 5 343 870
- US-A- 5 534 018
- US-A- 5 713 935
- US-A- 6 076 015

## Description

L'invention concerne les enregistreurs de signaux physiologiques, tels que les enregistreurs ambulatoires de type Holter assurant l'enregistrement de signaux électrocardiographiques sur une longue durée.

Ces enregistreurs comportent un boîtier externe, porté par le patient, auquel sont reliées des électrodes de recueil appliquées sur le corps de ce patient et reliées au boîtier par l'intermédiaire de câbles de liaison.

Lors d'un usage fréquent, en particulier dans le cas des enregistreurs de longue durée, ces câbles sont soumis à de nombreuses contraintes et finissent par se dégrader, cette dégradation pouvant aller jusqu'à rupture de la liaison électrique interne.

L'état des câbles de liaison doit être surveillé, ce qui peut être réalisé de façon automatique par une mesure d'impédance, en faisant circuler dans ce câble un microcourant de mesure injecté par l'entrée de signal sur laquelle est branché ce câble (le retour du courant s'effectuant par une autre électrode de mesure ou par l'électrode de masse). La tension mesurée sur l'entrée de signal permet alors de déterminer l'impédance de la ligne, à laquelle s'ajoutent l'impédance des électrodes et l'impédance du corps du patient entre les deux électrodes comprises dans la boucle de courant. On peut mettre en évidence les défauts de liaisons électriques internes aux câbles, ainsi que les contacts défectueux des électrodes (électrode mal collée, coupure interne, etc.) en mesurant ainsi, en place, l'impédance des différents câbles ECG.

Cette mesure d'impédance peut s'effectuer non seulement au moment de la mise en route de l'appareil après mise en place des électrodes, mais également pendant toute la durée de l'enregistrement : il est ainsi possible de détecter un décollement d'électrode, le débranchement d'un câble, etc. et de tenir compte de ce défaut pour l'enregistrement ou l'analyse du signal, ou même demander au porteur de l'appareil de rétablir lui-même la continuité en replaçant l'électrode ou en rebranchant un câble déconnecté.

Le microcourant de mesure d'impédance est de préférence de nature impulsionnelle, car un microcourant continu risquerait de provoquer une polarisation du couple électrode/peau, et un microcourant alternatif entraînerait une consommation d'énergie relativement importante en raison de sa fréquence élevée, de l'ordre de 100 kHz, qui doit être supérieure aux composantes du signal ECG (y compris les pics de stimulation d'un stimulateur cardiaque éventuellement implanté sur le patient), et des signaux qui doivent être rejetés.

Pour cette raison, les appareils les plus récents, tels que les modèles *Syneflash* et *Synesis* d'Ela Médical, mettent en oeuvre un tel procédé impulsionnel, dans lequel l'appareil injecte une petite quantité de courant (de l'ordre du microampère) pendant un temps très court (de l'ordre de la microseconde) pour mesurer l'impédance de la ligne. L'amplificateur sur lequel est branchée l'entrée amplifie l'écart de tension provoqué par l'injection de ce courant et retransmet cet écart au système de mesure numérique (convertisseur analogique/numérique et microcontrôleur) qui mémorise et traite l'information d'impédance, sans (théoriquement) altérer le signal recueilli. Un tel procédé est décrit de façon détaillée en particulier dans le EP-A-0 711 530 (ELA Medical.

Ce procédé présente cependant deux inconvénients, dans certaines situations.

En effet, lorsqu'une électrode est débranchée, le niveau de tension de l'entrée sur laquelle est injecté le courant est parasité (situation qui se traduit par un signal illisible ou par un parasitage à la fréquence du secteur), ou tombe à zéro. Si le niveau tombe à zéro, l'appareil reçoit en fait sur l'amplificateur le signal ECG de l'autre électrode. Il amplifie donc cet autre signal en mode monopolaire, c'est-à-dire par rapport à la masse du patient, donnant ainsi un signal qui apparaît comme très correct, bien que détecté comme anormal par le microcontrôleur. Dans cette situation, le système d'injection de courant présente alors un défaut majeur, tenant au fait que la charge injectée sur la ligne qui présente une haute impédance (du fait de la coupure ou du débranchement du câble) crée une tension qui se maintient longtemps du fait des condensateurs d'entrée du circuit de recueil (condensateurs qui servent pour le filtrage passif des hautes fréquences) : le signal ECG capté se trouve alors très fortement perturbé par des pics provoqués à chaque injection du microcourant.

Par ailleurs, dans le cas d'un patient porteur d'un stimulateur cardiaque, les pics de stimulation du dispositif implanté peuvent gêner la mesure d'impédance de l'enregistreur externe, et ce, de deux manières :
- si le pic de stimulation survient au même moment qu'est effectuée la mesure, il peut déformer considérablement la réponse et donner une valeur fausse de l'impédance ;
- lorsque le pic de tension provoqué par l'injection de courant est significatif, il peut être à tort interprété comme un pic de stimulation par l'enregistreur.

L'un des buts de la présente invention est de proposer un nouvel appareil de mesure impulsionnelle d'impédance adapté aux enregistrements de longue durée et qui réduise les divers inconvénients précités.

À cet effet, l'invention propose un procédé de test de continuité électrique d'une ligne du type général divulgué par le EP-A-0 711 530 precité, correspondant au préambule de la revendication 1. L'invention propose de mettre en oeuvre les étapes visées à la partie caractérisante de la revendication 1 pour réponse l'ensemble des problèmes particuliers exposés plus haut.

L'invention a également pour objet un enregistreur de signaux physiologiques comprenant des moyens pour la mise en oeuvre des étapes du procédé exposé ci-dessus, conformément à la revendication indépendante 12.

Des formes de mise en oeuvre avantageuses sont exposées aux sous-revendications 2-11 et 13-17.

On va maintenant décrire plus en détail un exemple de mise en oeuvre de l'invention, en référence aux dessins annexés.
La figure 1 est une vue schématique des circuits d'un boîtier d'enregistreur permettant la mise en oeuvre de la présente invention.
La figure 2 illustre les chronogrammes de l'impulsion de courant injectée et du signal de tension résultant recueilli, d'abord dans le cas d'une liaison électrique correcte, puis dans celui d'une liaison défectueuse.
La figure 3 est une vue partielle illustrant une variante de mise en oeuvre de la figure 1.
La figure 4 est une généralisation de la figure 1 à la mesure simultanée des impédances sur plusieurs entrées.
La figure 5 illustre la manière de sélectionner les points d'échantillonnage d'un signal ECG permettant d'intercaler la mesure d'impédance dans le processus courant d'échantillonnage du signal ECG.

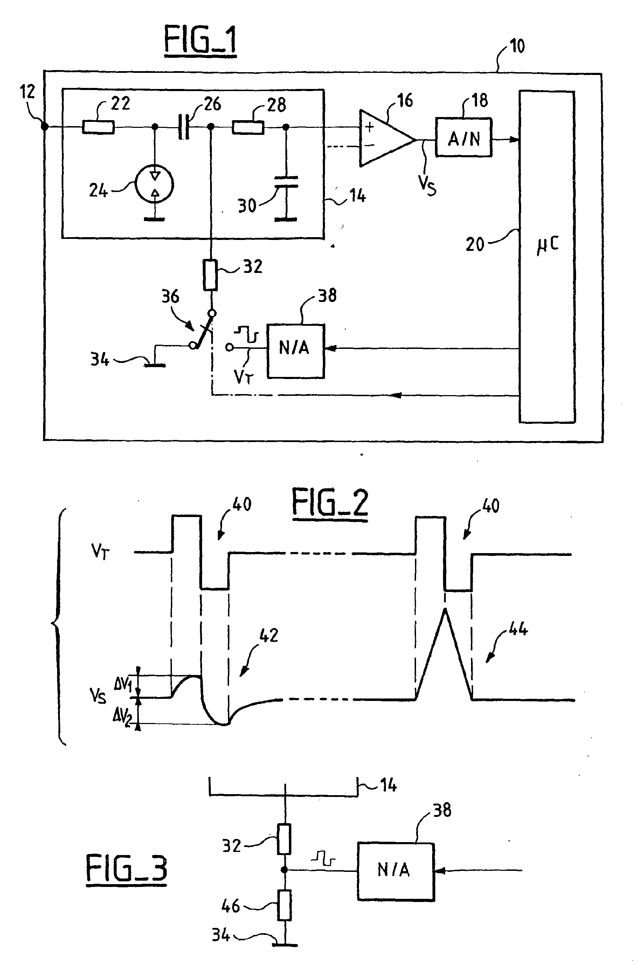

Sur la figure 1, on a référencé 10 le boîtier de l'appareil enregistreur, qui est pourvu d'entrées de signal dont une seule a été représentée en 12 (ces entrées sont typiquement au nombre de sept dans le cas d'un appareil d'enregistrement des ECG). Chaque entrée est raccordée à un câble de liaison (non représenté) dont l'autre extrémité est connectée à une électrode respective appliquée à un patient pour permettre le recueil des signaux physiologiques.

Chaque entrée de signal 12 comporte un circuit de protection 14 dont la sortie est reliée à l'une des entrées d'un amplificateur différentiel 16. L'autre entrée de cet amplificateur est reliée soit une autre entrée de signal (pour une mesure bipolaire) soit au potentiel de la masse (pour une mesure monopolaire).

La sortie de l'amplificateur 16 est, après conditionnement par un filtrage passe-haut et passe-bas, convertie par un convertisseur analogique/numérique 18 en un signal qui est appliqué à un microcontrôleur 20 assurant le traitement (filtrage, compression, enregistrement, etc.) de ce signal ainsi que celui des signaux issus des autres électrodes placées sur le patient.

Le circuit de protection 14 comporte une résistance série 22, de l'ordre de 100 kΩ, destinée à limiter le courant circulant dans le câble et par l'électrode. Un éclateur 24 assure une limitation en tension de l'entrée, de l'ordre de 40 V, pour prendre en compte notamment les chocs de défibrillation susceptibles d'être appliqués au patient et éviter que ces derniers ne détruisent les circuits d'entrée de l'enregistreur. Le circuit de protection 14 comporte également un condensateur de liaison 26 permettant de bloquer toute composante continue éventuelle, ainsi qu'un réseau RC 28, 30, permettant de filtrer les fréquences les plus élevées en amont de l'amplificateur différentiel 16.

Chaque entrée est également pourvue d'une résistance 32 de rappel à la masse, de valeur élevée (typiquement de l'ordre de 10 MΩ), montée entre la ligne de signal, d'une part, et la masse 34, d'autre part.

Pour la mesure d'impédance, l'invention propose, au lieu d'une liaison permanente à la masse de cette résistance de rappel 32, de relier le pied de celle-ci à un commutateur 36 qui la reliera soit à la masse (en position normale) soit à un générateur de tension ou de courant 38 (lors de la mesure d'impédance). La commutation est opérée sous la commande du microcontrôleur.

Pour des raisons pratiques, il est généralement plus simple d'utiliser pour le générateur 38 un générateur de tension, souvent disponible sur les microcontrôleurs sous forme d'un convertisseur numérique/analogique.

Plus précisément, pour la mesure d'impédance le générateur 38 fournit pendant un premier cycle de courte durée, par exemple 1 ms, une tension V_{T} d'une certaine polarité, puis pendant un second cycle de même durée, une tension de polarité inverse (cf. la première ligne du chronogramme de la figure 2, où la forme du signal correspondant est illustrée en 40).

Cette inversion des polarités permet d'annuler les charges dans la ligne, de sorte que la mesure du signal ECG ne sera plus perturbée, même avec une impédance de ligne élevée, par exemple du fait d'une électrode débranchée.

L'amplitude relative des créneaux positif et négatif de l'impulsion 40 peut être éventuellement optimisée, par apprentissage au niveau du microcontrôleur, de manière à ajuster le retour du signal résultant à la ligne de base en l'absence de câble, cet ajustement étant obtenu en introduisant une légère dissymétrie entre les deux créneaux pour compenser les différents décalages susceptibles d'être introduits dans la chaîne de mesure.

La valeur de l'impédance est calculée à partir des écarts de tension V_{S} mesurés en sortie de l'amplificateur à la fin du premier cycle (écart ΔV₁) et du second cycle (écart ΔV₂) (le signal V_{S} mesuré est illustré en 42 sur la deuxième ligne du chronogramme de la figure 2, dans le cas normal, c'est-à-dire d'une électrode non débranchée et un fil de liaison intact). Le pic de tension provoqué par la double injection de courant a dans ce cas une amplitude proportionnelle à l'impédance, et les écarts de tension ΔV₁ et ΔV₂ sont en valeur absolue approximativement égaux, aux erreurs de dissymétrie près.

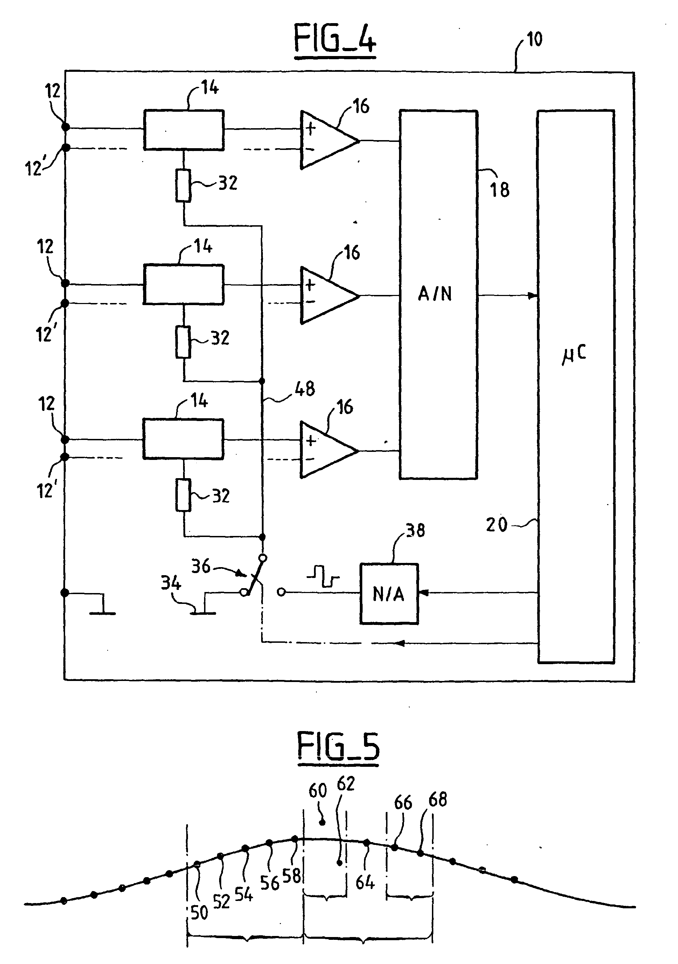

Pour optimiser le calcul de l'impédance, il est possible d'additionner les valeurs absolues des écarts de tension ΔV₁ et ΔV₂: comme on l'expliquera plus bas à propos de la figure 5, lorsque le signal ECG n'est pas nul, il présente des variations assimilables localement à une pente qui, si l'on considère que cette dernière est stable sur la courte période de la mesure d'impédance, altère les deux valeurs ΔV₁ et ΔV₂ en sens opposés ; le total de ces deux valeurs reste donc stable.

Dans le cas d'une impédance élevée, correspondant à une situation d'un câble débranché ou coupé, ou d'une électrode déconnectée, la réponse à l'impulsion de courant 40 est très sensiblement modifiée, comme illustré en 44 sur le second chronogramme de la figure 2 : la charge injectée sur la ligne à haute impédance lors du premier cycle de l'impulsion de courant 40 crée une tension élevée, que le microcontrôleur peut immédiatement détecter. Par ailleurs, l'injection de courant en sens inverse lors du second cycle de l'impulsion de courant 40 provoquera une annulation des charges stockées dans les diverses capacités d'entrées (charges qui ne se sont pas écoulées du fait de l'impédance élevée entre la borne de signal et la masse), permettant ainsi le retour du signal à la ligne de base, ce qui a pour effet de replacer l'ensemble du système, du point de vue des charges électriques, à son état antérieur.

La mesure d'impédance est décidée par le microcontrôleur à intervalles réguliers, par exemple quelques fois par minute ou quelques fois par seconde.

La mesure d'impédance prendra en compte les divers composants du circuit 14 de protection à l'encontre des interférences hautes et basses fréquences. Les composants ne gênent pas les mesures d'impédance, mais il doivent être pris en compte dans ces dernières. Ainsi, la résistance série 22 placée en entrée pour limiter le courant pouvant circuler dans la ligne lorsqu'une tension élevée est appliquée à une électrode, par exemple dans le cas d'un choc de défibrillation, vient s'ajouter à la mesure d'impédance. Quant au condensateur 26 chargé de couper les très basses fréquences, il n'intervient pas dans le calcul du fait qu'il transmet intégralement le courant de mesure à la ligne ; il présente au surplus l'avantage de supprimer d'éventuelles composantes continues susceptibles de diminuer la précision des courants injectés par le générateur de tension.

Pour fiabiliser la mesure d'impédance, l'enregistreur doit éviter de prendre en compte des valeurs aberrantes. Pour cela il peut pratiquer plusieurs mesures successives pour n'en garder qu'une seule, par exemple la moyenne ou la médiane des mesures effectuées ; on élimine de cette façon le risque d'enregistrer une mesure aberrante si, par exemple, un artefact haute fréquence apparaît pendant le cycle de mesure d'impédance. Ainsi, si le dispositif effectue par exemple dix mesures pendant un intervalle d'une minute, il enregistre chaque minute la valeur médiane de ces dix mesures : la valeur de l'impédance est donc mise à jour une fois par minute.

Diverses variantes et améliorations de l'invention peuvent être envisagées.

Ainsi, il est possible de tester deux lignes simultanément par des signaux en opposition de phase. Le courant circulant alors entre deux électrodes, on élimine le risque de fausse mesure si l'électrode de masse est décollée (le bilan de charge final reste nul).

D'autre part, comme illustré figure 3, il est possible de supprimer le commutateur 36, en laissant connecté en permanence le générateur de tension 38 au pied de la résistance 32, avec interposition d'une résistance 46 entre la résistance 32 et la masse 34. Entre les phases de mesure, au lieu de basculer le commutateur 36 sur la masse 34 comme dans le mode de réalisation précédent, le microcontrôleur impose au générateur 38 une tension de sortie nulle, ce qui revient à mettre le pied de la résistance 32 au potentiel de la masse. Cette solution, purement statique, permet d'éviter le recours à un interrupteur commandé 36 ; elle présente cependant l'inconvénient de nécessiter un fonctionnement permanent du générateur de tension, qui consomme donc en permanence un certain courant, du fait du réseau de résistances que ce dernier comporte (à la différence du mode de réalisation de la figure 1 où le commutateur 36 permet d'arrêter complètement le générateur en dehors des cycles de mesure de l'impédance, faisant ainsi l'économie du courant d'alimentation du générateur 38).

Par ailleurs, il est possible de réaliser la mesure d'impédance simultanément sur plusieurs amplificateurs.

Ainsi, comme illustré figure 4, l'enregistreur 10 comporte une pluralité d'entrées telles que 12, reliée chacune, via un circuit de protection 14 correspondant, à l'entrée positive d'un amplificateur respectif 16. De la même façon, des entrées 12' sont reliées à l'entrée négative de ces mêmes amplificateurs 16. Chacune des entrées positives 12 est reliée par sa résistance de pied 32 respective, via une ligne commune 48, à un commutateur unique 36 (ou à une résistance 46 unique, dans le cas de la variante de la figure 3). De la même façon, les entrées négatives sont branchées sur un autre commutateur commun. Les lignes sont testées ainsi en deux temps : les entrées positives en premier, et les entrées négatives en dernier, par exemple.

La mesure de l'impédance étant réalisée en un temps très court, il est possible de réaliser cette mesure sans interrompre le recueil du signal ECG. La figure 5 illustre la manière de procéder correspondante.

Pour pouvoir mesurer l'impédance, il est nécessaire de disposer d'un amplificateur ayant une constante de temps suffisamment courte devant les cycles de mesure. Cette constante doit être en particulier plus courte que celle qui est utilisée couramment pour un échantillonnage à 200 s⁻¹ (soit environ 10 ms).

L'invention propose d'associer une constante de temps plus courte, par exemple 2 ms au lieu de 10 ms, avec un échantillonnage plus rapide, par exemple à 1 kHz, en moyennant ensuite le signal sur plusieurs points.

La figure 5 illustre un signal ECG avec les divers points d'échantillonnage successifs tels que 50 ... 58 et 60 ... 68.

La valeur du signal ECG est obtenue en moyennant cinq points d'échantillonnage successifs 50, 52, 54, 56 et 58. Les échantillons étant numérisés chaque milliseconde, cette opération donne 1000/5 = 200 points par seconde (fréquence qui est une valeur de fréquence d'acquisition couramment utilisée dans les enregistreurs existants, qui permettent un échantillonnage à des fréquences généralement comprise entre 128 et 256 Hz).

Au moment d'une mesure d'impédance, l'injection de la double impulsion de courant va se traduire par deux points d'échantillonnage 60 et 62 s'écartant de la ligne de base, et correspondant aux amplitudes ΔV₁ et ΔV₂ du signal en réponse illustré figure 2. Ces deux points 60 et 62 seront utilisés pour la mesure d'impédance et, sur les cinq échantillons successifs, il restera au moins deux points 66, 68 pour délivrer une valeur de signal ECG (le point 64 qui suit immédiatement les points 60 et 62 n'est pas conservé pour le calcul de la valeur moyenne, par précaution).

De ce fait, la détermination du signal ECG ne se trouvera pas, ou du moins très peu, altérée pendant la phase de mesure d'impédance : la seule différence étant qu'au cours de ce cycle particulier l'évaluation du signal ECG se fera par moyennage de deux points, au lieu de cinq dans le cas normal - mais la mesure du signal ECG ne sera pas interrompue, même pendant un seul cycle, du fait de la mesure d'impédance.

Cette manière de procéder n'est bien entendu pas limitative, et d'autres variantes peuvent être envisagées, par exemple la mesure de trois ou quatre points par cycle, au lieu de cinq, en ne retenant que le dernier point comme valeur du signal ECG dans le cas d'un cycle incluant une mesure d'impédance.

On va maintenant considérer le cas particulier d'un patient porteur d'un stimulateur cardiaque.

Dans ce cas, les pics de stimulation émis par le stimulateur viennent perturber le fonctionnement de la mesure d'impédance par l'enregistreur.

En effet, ces pics sont d'une amplitude et d'une durée du même ordre de grandeur que les variations de tension liées à la mesure d'impédance ; le circuit de mesure peut donc confondre le signal recueilli lors de la mesure d'impédance (le signal illustré sur la seconde ligne de la figure 2) avec un pic de stimulation.

Jusqu'à présent, cette difficulté était contournée en supprimant toute mesure d'impédance par les enregistreurs portés par des patients dotés d'un stimulateur cardiaque.

L'invention propose de remédier à cette difficulté, en proposant deux méthodes autorisant la mesure d'impédance par l'enregistreur même lorsque le patient porte un stimulateur cardiaque.

La *première méthode* consiste à synchroniser le cycle de mesure d'impédance sur les pics de stimulation reconnus.

Pour cela, l'enregistreur recherche la présence de pics (analyse du signal à 1000 Hz par le logiciel ou par un circuit spécifique), et délivre des fenêtres temporelles placées après les pics (10 ms par exemple), au cours desquels il sera certain que le stimulateur n'émettra pas d'autres impulsions de stimulation. Lorsque l'enregistreur veut effectuer la mesure d'impédance, il attend une fenêtre de mesure et opère la mesure comme précédemment sans risquer d'être perturbé.

Dans le cas où aucun pic n'est détecté dans le signal ECG pendant une durée relativement importante (quelques secondes), l'enregistreur considère que le stimulateur est en mode inhibé (mode "sentinelle") et qu'il peut donc lancer sa mesure d'impédance ; il reste cependant un risque (faible) de se trouver en synchronisme avec un pic, si une stimulation se déclenche à ce moment.

La *seconde méthode* consiste à reconstruire le signal à 1000 Hz : lors d'une mesure antérieure, la réponse à l'impulsion de courant est mémorisée. En fonctionnement normal, à chaque mesure d'impédance, le signal ECG est recueilli et, à l'aide d'un algorithme de soustraction, cette réponse préalablement enregistrée est soustraite du signal réel. On obtient ainsi un signal débarrassé des artefacts de mesure d'impédance, et sur lequel on peut rechercher les pics de stimulation (la réponse propre à l'injection du signal de mesure est obtenue à partir des cycles précédents, et éventuellement suivants).

## Revendications

1. Un enregistreur (10) de signaux physiologiques, notamment un enregistreur Holter de signaux ECG, comprenant des moyens de test de la continuité électrique d'une ligne comportant un câble de liaison reliant une électrode externe placée sur un patient à une borne de signal (12) de l'enregistreur, cette continuité étant susceptible d'être mise en défaut par un débranchement ou une coupure du câble, ou par une déconnexion ou un décollement de l'électrode externe,
ces moyens comprenant :
- des moyens (20, 32, 36, 38) pour générer une impulsion de courant (40) et appliquer cette impulsion sur ladite ligne,
- des moyens (16, 18, 20) pour mesurer la variation de tension résultante (42, 44) sur la borne de signal pendant la durée d'application de l'impulsion de courant, et
- des moyens pour déterminer l'impédance de la ligne à partir de la tension ainsi mesurée,
**caractérisé en ce que** :
- les moyens (20, 32, 36, 38) pour générer l'impulsion de courant (40) sont des moyens aptes à générer une impulsion biphasique comprenant deux cycles successifs de polarités inverses, les durées et les amplitudes de ces deux cycles étant choisis de manière à définir des charges respectives sensiblement égales et de signes contraires.

2. L'enregistreur de la revendication 1, dans lequel les moyens de mesure de la variation de tension mesurent la variation de tension à la fin du premier cycle (ΔV₁) et à la fin du second cycle (ΔV₂) de l'impulsion de courant.

3. L'enregistreur de la revendication 2, dans lequel les moyens de mesure de la variation de tension additionnent les valeurs absolues des variations de tension mesurées à la fin du premier cycle (ΔV₁) et à la fin du second cycle (ΔV₂) de l'impulsion de courant.

4. L'enregistreur de la revendication 2, dans lequel les moyens de mesure de la variation de tension opèrent de façon concomitante au recueil du signal ECG, et dans lequel il est prévu des moyens pour échantillonner ledit signal ECG à une fréquence procurant une pluralité d'échantillons (50, 52, 54, 58 ; 60, 62, 64, 68) par cycle de mesure, avec au moins deux échantillons (60, 62) pour la mesure de la variation de tension à la fin du premier cycle et à la fin du second cycle de l'impulsion de courant, et au moins un échantillon (50, 52, 54, 58 ; 66, 68) pour la mesure du niveau du signal ECG.

5. L'enregistreur de la revendication 4, dans lequel ledit signal ECG est échantillonné à une fréquence procurant une pluralité d'échantillons (50, 52, 54, 58 ; 66, 68) pour la mesure du niveau du signal ECG, et dans lequel la mesure du signal ECG est effectuée par moyennage de cette pluralité d'échantillons.

6. L'enregistreur de la revendication 4, dans lequel ledit signal ECG est échantillonné à une fréquence procurant au moins deux échantillons (60, 62) pour la mesure la mesure de la variation de tension à la fin du premier cycle et à la fin du second cycle de l'impulsion de courant, avec éventuellement un échantillon intercalaire (64), et au moins un échantillon (66, 68) pour la mesure du niveau du signal ECG.

7. L'enregistreur de la revendication 1, comportant en outre des moyens d'élimination des pics de stimulation produits par un stimulateur cardiaque implanté chez le patient et mêlés au signal ECG recueilli.

8. L'enregistreur de la revendication 7, dans lequel les moyens d'élimination des pics de stimulation mêlés au signal ECG comportent des moyens de détection préalable des pics de stimulation et de définition d'une fenêtre temporelle post-stimulation au cours de laquelle seront mis en oeuvre lesdits moyens de test de la continuité électrique de la ligne.

9. L'enregistreur de la revendication 7, dans lequel les moyens d'élimination des pics de stimulation mêlés au signal ECG comportent des moyens de mémorisation préalable de la variation de tension sur la borne de signal pendant la durée d'application de l'impulsion de courant, et des moyens de soustraction de cette variation mémorisée lors de la détection d'un pic de stimulation.

10. L'enregistreur de la revendication 1, dans lequel lesdits moyens de test de la continuité électrique de la ligne sont des moyens opérant de manière itérative, et dans lequel l'enregistreur comprend en outre des moyens d'évaluation et de mémorisation périodiques d'une valeur d'impédance à partir d'une pluralité de mesures d'impédance antérieures.

11. L'enregistreur de la revendication 10, dans lequel lesdits moyens d'évaluation et de mémorisation périodiques d'une valeur d'impédance recherchent une médiane de ladite pluralité de mesures antérieures.

12. L'appareil de la revendication 1, dans lequel les moyens pour générer l'impulsion de courant comprennent un générateur (38) de tension ou de courant piloté par un microcontrôleur (20) de l'enregistreur, la tension ou le courant de sortie de ce générateur étant appliqué au pied d'une résistance (32) de rappel à la masse couplée par ailleurs à la borne de signal (12), et dans lequel il est prévu des moyens (36, 46) pour mettre au potentiel de la masse (34) le pied de la résistance de rappel en dehors des périodes d'application du courant ou de la tension de sortie du générateur.

13. L'appareil de la revendication 12, dans lequel les moyens pour mettre au potentiel de la masse le pied de la résistance de rappel en dehors des périodes d'application du courant ou de la tension de sortie du générateur comprennent un commutateur (36) commandé sélectivement entre un état de recueil du signal ECG, où le pied de la résistance de rappel (32) est relié au potentiel de la masse (34), et un état de mesure d'impédance, où le pied de la résistance de rappel (32) est relié à la sortie du générateur (38).

14. L'appareil de la revendication 12, dans lequel les moyens pour mettre au potentiel de la masse le pied de la résistance de rappel en dehors des périodes d'application du courant ou de la tension de sortie du générateur comprennent une résistance (46) intercalée entre la masse (34) et le pied de la résistance de rappel (32), le courant ou la tension de sortie du générateur (38) étant forcés à une valeur nulle en dehors desdites périodes d'application.

15. L'appareil de la revendication 12, comportant une pluralité de bornes de signal (12, 12, 12), chacune pourvue d'une résistance de rappel respective (32, 32, 32), dans lequel la tension ou le courant de sortie du générateur (38) est appliqué simultanément, soit aux entrées positives soit aux entrées négatives, aux pieds de ces diverses résistances de rappel, et dans lequel les moyens (36, 46) de mise au potentiel de la masse en dehors des périodes d'application sont des moyens communs opérant simultanément sur lesdites résistances de rappel.

16. L'appareil de la revendication 12, comportant une pluralité de bornes de signal (12, 12, 12), chacune pourvue d'une résistance de rappel respective (32, 32, 32), dans lequel la tension ou le courant de sortie du générateur (38) est appliqué indépendamment aux pieds de ces diverses résistances de rappel, et dans lequel les moyens (36, 46) de mise au potentiel de la masse en dehors des périodes d'application sont des moyens comportant une pluralité de commutateurs, avec un commutateur par bor ne de signal.

## Claims

1. Apparatus (10) for recording physiological signals, in particular Holster-apparatus for recording ECG-signals, comprising means for testing an electric continuity of a line comprising a connection cable connecting an external electrode placed on a patient to a signal terminal (12) of the recording apparatus, said continuity being susceptible to failure due to a disconnection or disruption of the cable, or due to a disconnection or detachment of the external electrode, said means including:
- means (20, 32, 36, 38) for generating a current impulse (40) and applying said impulse to said line,
- means (16, 18, 20) for measuring a voltage variation (42, 44) resulting on the signal terminal during the duration of application of the current impulse, and
- means for determining the impedance of the line based upon said measured voltage,
**characterised in that**:
- said means (20, 32, 36, 38) for generating the current impulse (40) is a means adapted for generating a biphasic impulse including two successive cycles of opposite polarities, the duration and amplitude of these two cycles being selected to define approximately equal respective loads of contrary signs.

2. The recording apparatus of claim 1, wherein the means for measuring the voltage variation measures the voltage variation at the end of the first cycle (ΔV₁) and at the end of the second cycle (ΔV₂) of the current impulse.

3. The recording apparatus of claim 2, wherein the means for measuring the voltage variation adds the absolute values of the voltage variations measured at the end of the first cycle (ΔV₁) and at the end of second cycle (ΔV₂) of the current impulse.

4. The recording apparatus of claim 2, wherein the means for measuring the voltage variation concomitantly performs the collection of the ECG-signal, and wherein there is provided a means for sampling said ECG-signal at a frequency delivering a plurality of samples (50, 52, 54, 58; 60, 62, 64, 68) per measurement cycle, with at least two samples (60, 62) for the measurement of the voltage variation at the end of the first cycle and the end of the second cycle of the current impulse, and at least one sample (50, 52, 54, 58; 66, 68) for the measurement of the ECG-signal level.

5. The recording apparatus of claim 4, wherein the ECG-signal is sampled at a frequency delivering a plurality of samples (50, 52, 54, 58; 66, 68) for the measurement of the ECG-signal level, and wherein measuring the ECG-signal is effected by averaging said plurality of samples.

6. The recording apparatus of claim 4, wherein the ECG-signal is sampled at a frequency delivering at least two samples (60, 62) for the measurement of the voltage variation at the end of the first cycle and at the end of the second cycle of the current impulse, with possibly an intervening sample (64), and at least one sample (66, 68) for the measurement of the ECG-signal level.

7. The recording apparatus of claim 1, furthermore comprising means for eliminating stimulation peaks produced by a cardiac pacemaker implanted in the patient and mixed to the collected ECG-signal.

8. The recording apparatus of claim 7, wherein the means for eliminating the stimulation peaks mixed to the ECG-signal comprises means for preliminary detection of the stimulation peaks and definition of a temporal post-stimulation window, during which said means for testing the electric continuity of the line will be put into practice.

9. The recording apparatus of claim 7, wherein said means for eliminating the stimulation peaks mixed to the ECG-signal comprises means for preliminary memorisation of the voltage variation on the signal terminal during the duration of application of the current impulse, and means for subtracting said memorized variation at detection of a stimulation peak.

10. The recording apparatus of claim 1, wherein said means for testing the electric continuity of the line is a means operating in a reiterative fashion, and wherein the recording apparatus furthermore comprises means for periodically evaluating and memorizing an impedance value based upon a plurality of previous impedance measurements.

11. The recording apparatus of claim 10, wherein said means for periodically evaluating and memorizing an impedance value seeks a median of said plurality of previous measurements.

12. The apparatus of claim 11, wherein the means for generating the current impulse comprises a voltage or current generator (38) controlled by a microcontroller (20) of the recording apparatus, the voltage or current output of said generator being applied to the base of a pull-down to ground resistor (32) being otherwise coupled to the signal terminal (12), and wherein there is provided a means (36, 46) for placing the base of the pull-down resistor at the potential of the ground (34) outside the periods of application of the output current or voltage of the generator.

13. The apparatus of claim 12, wherein the means for placing the base of the pull-down resistor at ground potential outside the periods of application of the output current or voltage of the generator comprises a switch (36) selectively controlled between a state for collecting an ECG-signal, wherein the base of the pull-down resistor (32) is connected to the potential of the ground (34), and an impedance measurement state, wherein the base of the pull-down resistor (32) is connected to the output of the generator (38).

14. The apparatus of claim 12, wherein the means for placing the base of the pull-down resistor at ground potential outside the periods of application of the output current or voltage of the generator comprises a resistor (46) inserted between the ground (34) and the base of the pull-down resistor (32), wherein the current or the voltage output of the generator (38) is forced to a zero value other than during said application periods.

15. The apparatus of claim 12, comprising a plurality of signal terminals (12, 12, 12), each having a corresponding pull-down resistor (32, 32, 32), wherein the voltage or the current output of the generator (38) is applied simultaneously, either at the positive inputs or at the negative inputs, at the bases of said various pull-down resistors, and wherein the means (36, 46) for placing at ground potential outside the periods of application is a common means operating simultaneously on said pull-down resistors.

16. The apparatus of claim 12, comprising a plurality of signal terminals (12, 12, 12), each having a corresponding pull-down resistor (32, 32, 32), wherein the voltage or the current output of the generator (38) is applied independently to the bases of said various pull-down resistors, and wherein the means (36, 46) for placing at ground potential outside the periods of application is a means comprising a plurality of switches, with one switch per signal terminal.

## Patentansprüche

1. Vorrichtung (10) zur Aufnahme von physiologischen Signalen, insbesondere eine Holter-Vorrichtung zur Aufnahme von EKG-Signalen, mit Mitteln zum Prüfen des elektrischen Durchgangs einer Leitung, die ein Verbindungskabel umfasst, welches eine an einem Patienten angeordnete Außenelektrode mit einem Signalanschluss (12) der Aufnahmevorrichtung verbindet, wobei dieser Durchgang wegen eines Herausziehens oder eines Bruchs des Kabels, oder wegen eines Entfernens oder Ablösens der Au-βenelektrode fehlerhaft sein kann,
wobei diese Mittel folgendes umfassen:
- Mittel (20, 32, 36, 38) zum Erzeugen eines Stromimpulses (40) und zur Anwendung dieses Impulses auf die Leitung,
- Mittel (16, 18, 20) zur Messung der resultierenden Spannungsänderung (42, 44) am Signalanschluss während der Anwendungsdauer des Stromimpulses, und
- Mittel zur Bestimmung der Impedanz der Leitung anhand der so gemessenen Spannung,
**dadurch gekennzeichnet, dass:**
- die Mittel (20, 32, 36, 38) zur Erzeugung des Stromimpulses (40) Mittel sind, die geeignet sind, einen zweiphasigen Impuls zu erzeugen, mit zwei aufeinander folgenden Zyklen umgekehrter Polarität, wobei die Dauer und die Amplitude dieser zwei Zyklen so gewählt sind, um jeweilige im wesentlichen gleiche Ladungen von entgegen gesetztem Vorzeichen zu definieren.

2. Aufnahmevorrichtung nach Anspruch 1, bei welcher die Mittel zur Messung der Spannungsänderung die Spannungsänderung am Ende des ersten Zyklus (ΔV₁) und am Ende des zweiten Zyklus (ΔV₂) des Stromimpulses messen.

3. Aufnahmevorrichtung nach Anspruch 2, bei welcher die Mittel zur Messung der Spannungsänderung die Absolutwerte der am Ende des ersten Zyklus (ΔV₁) und am Ende des zweiten Zyklus (ΔV₂) des Stromimpulses gemessenen Spannungsänderungen addieren.

4. Aufnahmevorrichtung nach Anspruch 2, bei welcher die Mittel zur Messung der Spannungsänderung begleitend die Aufnahme des EKG-Signals vornehmen, und bei welcher Mittel zur Abtastung des EKG-Signals mit einer Frequenz vorgesehen sind, die eine Mehrzahl von Abtastwerten (50, 52, 54, 58; 60, 62, 64, 68) pro Messzyklus liefert, mit mindestens zwei Abtastwerten (60, 62) für die Messung der Spannungsänderung am Ende des ersten Zyklus und am Ende des zweiten Zyklus des Stromimpulses, und wenigstens einem Abtastwert (50, 52, 54, 58; 66, 68) für die Messung des Niveaus des EKG-Signals.

5. Aufnahmevorrichtung nach Anspruch 4, bei welcher das EKG-Signal mit einer Frequenz abgetastet wird, die eine Mehrzahl von Abtastwerten (50, 52, 54, 58; 66, 68) zur Messung des Niveaus des EKG-Signals liefert, und bei welcher die Messung des EKG-Signals durch Mittlung dieser Mehrzahl an Abtastwerten erfolgt.

6. Aufnahmevorrichtung nach Anspruch 4, bei welcher das EKG-Signal mit einer Frequenz abgetastet wird, die wenigstens zwei Abtastwerte (60, 62) zur Messung der Spannungsänderung am Ende des ersten Zyklus und am Ende des zweiten Zyklus des Stromimpulses liefert, eventuell mit einem dazwischen liegenden Abtastwert (64), und wenigstens einen Abtastwert (66, 68) zur Messung des Niveaus des EKG-Signals.

7. Aufnahmevorrichtung nach Anspruch 1, weiterhin mit Mitteln zur Entfernung der Stimulationsspitzen, die durch einen beim Patienten implantierten Herzschrittmacher erzeugt werden und dem aufgenommenen EKG-Signal beigemischt sind.

8. Aufnahmevorrichtung nach Anspruch 7, bei welcher die Mittel zur Entfernung der dem EKG-Signal beigemischten Stimulationsspitzen Mittel zur Vorerfassung der Stimulationsspitzen und zur Definition eines poststimulativen Zeitfensters umfassen, während welchem die Mittel zum Prüfen des elektrischen Durchgangs der Leitung eingesetzt werden.

9. Aufnahmevorrichtung nach Anspruch 7, bei welcher die Mittel zur Entfernung der dem EKG-Signal beigemischten Stimulationsspitzen Mittel zur Vorspeicherung der Spannungsänderung am Signalanschluss während der Anwendungsdauer des Stromimpulses umfassen, sowie Mittel zum Abziehen dieser gespeicherten Änderung bei der Erfassung einer Stimulationsspitze.

10. Aufnahmevorrichtung nach Anspruch 1, bei welcher die Mittel zum Prüfen des elektrischen Durchgangs der Leitung Mittel sind, die iterativ arbeiten, und bei welcher die Aufuahmcvorrichtung weiterhin Mittel zur Schätzung und zur periodischen Speicherung eines Impedanzwerts anhand einer Mehrzahl von vorhergehenden Impedanzmessungen umfasst.

11. Aufnahmevorrichtung nach Anspruch 10, bei welcher die Mittel zur Schätzung und zur periodischen Speicherung eines Irnpedanzwerts einen Median der Mehrzahl an vorhergehenden Messungen suchen.

12. Vorrichtung nach Anspruch 1, bei welcher die Mittel zur Erzeugung des Stromimpulses einen durch einen Mikrocontroller (20) der Aufnahmevorrichtung gesteuerten Spannungs- oder Stromgenerator (38) umfassen, wobei die Ausgangsspannung oder -stromstärke diese Generators an den Anschluss eines Masse-Rückstellwiderstands (32) angelegt wird, der im übrigen an den Signalanschluss (12) gekoppelt ist, und bei welcher Mittel (36, 46) vorgesehen sind, um den Anschluss des Rückstellwiderstands außerhalb der Zeiträume der Anwendung der Ausgangsstromstärke oder - spannung des Generators auf das Potential der Masse (34) zu bringen.

13. Vorrichtung nach Anspruch 12, bei welcher die Mittel, um den Anschluss des Rückstellwiderstands außerhalb der Zeiträume der Anwendung der Ausgangsstromstärke oder -spannung des Generators auf das Potential der Masse zu bringen, einen Schalter (36) umfassen, der selektiv zwischen einem Zustand der Aufnahme des EKG-Signals, bei welchem der Anschluss des Rückstellwiderstands (32) mit dem Potential der Masse (34) verbunden ist, und einem Zustand der Impedanzmessung, bei welchem der Anschluss des Rückstellwiderstands (32) mit dem Ausgang des Generators (38) verbunden ist, gesteuert wird.

14. Vorrichtung nach Anspruch 12, bei welcher die Mittel, um den Anschluss des Rückstellwiderstands außerhalb der Zeiträume der Anwendung der Ausgangsstromstärke oder -spannung des Generators auf das Potential der Masse zu bringen, einen Widerstand (46) umfassen, der zwischen der Masse (34) und dem Anschluss des Rückstellwiderstands (32) eingefügt ist, wobei die Ausgangsstromstärke oder -spannung des Generators (38) außerhalb der Anwendungszeiträume auf Null gesetzt wird.

15. Vorrichtung nach Anspruch 12, mit einer Mehrzahl Signalanschlüssen (12, 12, 12), die jeder mit einem jeweiligen Rückstellwiderstand (32, 32, 32) versehen sind, bei welcher die Ausgangsspannung oder -stromstärke des Generators (38) gleichzeitig, entweder an den positiven Eingängen oder an den negativen Eingängen, an die Anschlüsse dieser diversen Rückstellwiderstände angelegt wird, und bei welcher die Mittel (36, 46) zum außerhalb der Anwendungszeiträume auf das Potential der Masse Bringen gemeinsame Mittel sind, die gleichzeitig auf die Rückstellwiderstände einwirken.

16. Vorrichtung nach Anspruch 12, mit einer Mehrzahl Signalanschlüssen (12, 12, 12), die jeder mit einem jeweiligen Rückstellwiderstand (32, 32, 32) versehen sind, bei welcher die Ausgangsspannung oder -stromstärke des Generators (38) unabhängig an die Anschlüsse dieser diversen Rückstellwiderstände angelegt wird, und bei welcher die Mittel (36, 46) zum außerhalb der Anwendungszeiträume auf das Potential der Masse Bringen Mittel sind, die eine Mehrzahl Schalter umfassen, mit einem Schalter pro Signalanschluss.
